# EUROPEAN PATENT APPLICATION

(11) **EP 0 971 032 A1**
(43) Date of publication of application: **12.01.2000**
(21) Application number: 98938938.2
(22) Date of filing: 21.08.1998
(51) Int. Cl.: C12N 1/14, C12N 1/20

(54) **GROWTH PROMOTING MATERIAL FOR USEFUL MICROORGANISMS AND PROCESS FOR PRODUCING THE SAME**

(30) Priority: 21.08.1997 JP 22529697
(71) Applicant: NICHIMO COMPANY LIMITED, Tokyo 100-0004 (JP)
(72) Inventor: TAKEBE, Minoru, Tokyo 100-0004 (JP); MORIMOTO, Masakazu, Tokyo 100-0004 (JP)
(74) Representative: Ouzman, Beverley Nicola Claire
(86) International application number: JP9803719
(87) International publication number: WO9910473

(57) **Abstract**

The beneficial microorganism propagation-promoting material and a process for preparing the same of the present invention are a beneficial microorganism propagation-promoting material that uses a product whose source is grains and that can promote the propagation of beneficial microorganisms that are effective in maintaining the health of a living being when it is being reared and serves as a nutrient that is superior in terms of hygiene, is easy to produce, and is inexpensive to produce, as well as a process with which beneficial microorganisms can be simultaneously propagated as the beneficial microorganism propagation-promoting material is produced. There has never been a product like the present invention. The present invention is mainly characterized in that grains are inoculated with koji mold to effect koji preparation, and the resultant from this koji preparation is hydrolyzed to obtain the beneficial microorganism propagation-promoting material with the above-described excellent effects.

## Description

### Technological Field

The present invention relates to a beneficial microorganism propagation-promoting material and a process for preparing the same, and more particularly, to a beneficial microorganism propagation-promoting material obtained from a product whose starting material is grains and to a process for preparing the same

The term Agrains≅ used in the present invention means soybeans, rice, wheat, corn, and their scraps, etc.; and at least one type of these grains is used as the starting material base. Products whose starting materials are grains means food products (for instance, tofu and soybean milk, etc.), livestock feed, feed for fisheries, etc. which incude grans as the starting materials.

The term Aorganic microorganism propagation-promoting material≅ used in the present invention means a material that promotes propagation of beneficial microorganisms (Eumycetes, such as yeast, etc., lactic acid bacteria, bifidobacteria and other beneficial bacteria) that are effective maintaining the health of a living being as it is being reared.

### Prior Art

In general, there is a strong demand for products whose starting materials are grains, and there are a variety of such products are being proposed.

Refined sake, vinegar, soy, miso, etc. are brewed products whose source is grains and which are produced using koji mold.

Nevertheless, almost all of these are delicacy foods, and it is safe to say thus far that there has been no such foods that can be consumed in large quantities for nutrition or health maintenance.

More specifically, koji mold has been used in brewing mainly for saccharification of grains to produce refined sake, shochu (Japanese traditional distilled liquor), vinegar, etc., and for the decomposition of proteins in the production of soy and miso. It is difficult to obtain these brewed products from koji mold only, and such products are usually made by using lactic acid bacteria and yeast, etc. as well, which means that the process uses the properties of koji mold. In other words, it can be said that these products can be produced because the koji mold is compatible and has the ability to coexist with lactic acid bacteria, yeast, etc. Lactic acid bacteria and yeast act to prevent the propagation of putrefying bacteria during the relatively long fermentation period of refined sake, soy sauce and miso, etc. Lactic acid bacteria propagate to produce lactic acid, and yeast produces alcohol under anaerobic conditions, so that each one of the above-described products can be produced.

The relationship of lactic acid bacteria and the brewed products of refined sake, miso and soy sauce, which use this koji mold, will be described in detail.

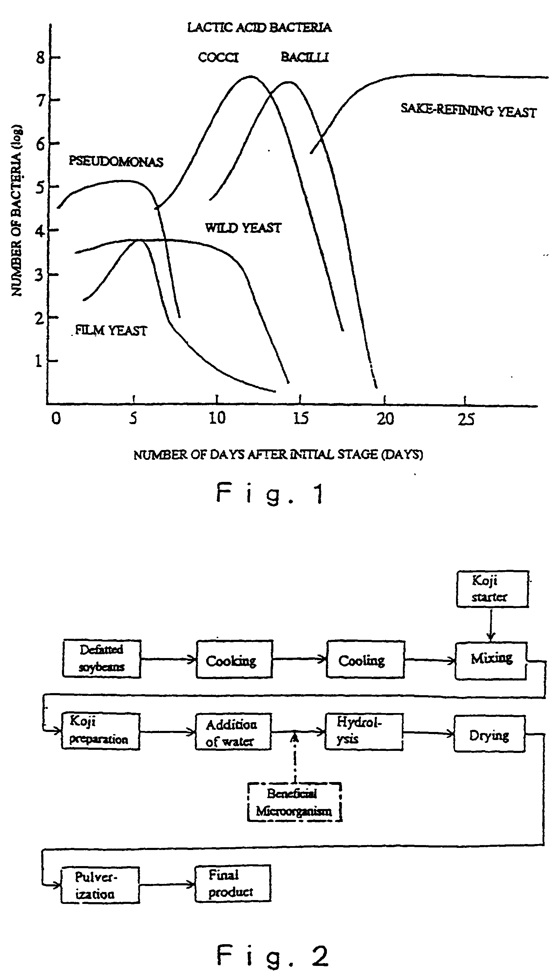

First, in methods for producing refined sake, lactic acid produced by lactic acid bacteria is used in order to cause the growth of large quantities of only sake-refining yeast which has a strong activity in the initial stage. Such methods include the traditional method that uses lactic acid bacteria naturally found in the sake (Aki moto≅ and Ayamahai moto≅) and the method in which lactic acid is added at the beginning (Asokujo moto≅). When Figure 1 is explained based on the yamahai moto, first, low-temperature nitric acid-reducing bacteria (*Pseudomonas*) are propagated in the sake yeast to which the active sake-refining yeast has been introduced at a low temperature of approximately 8°C, thus producing nitrous acid (approximately one week, 10 ppm). Thereafter the number of bacteria decreases. Next, the lactic acid bacteria propagate to produce lactic acid. The Lactic acid bacteria first propagate as cocci and then propagate as bacilli. During this time, the film yeast and wild yeast, etc. die due to the high sugar concentration, the reduction in pH attributed to the nitrous acid and lactic acid, the low temperature, etc. Microorganisms other than lactic acid bacteria are not detectable approximately two weeks after adding the sake yeast, thus creating optimum conditions for cultivation of the sake yeast. Superior refined sake yeast is inoculated at this time. The remaining lactic acid bacteria are killed by the alcohol that is produced when the fermentation of the sake yeast becomes active. As a result, sake yeast in which large amounts of the better inoculated sake-refining yeast exist is obtained.

On the other hand, lactic acid bacteria referred to as Ahiochii≅ bacteria tend to grow an alcohol component and changes, including turbidity, an increase in acids; and changes in smell, etc. occur. Therefore, this should be regarded as a problem. It is a known fact that Ahiochii≅ bacteria are lactic acid bacteria that requires Ahiochii≅ acid (mevalonic acid). This mevalonic acid has been shown to accumulate with the propagation of bacteria during the process of koji preparation by koji mold, such as *Aspergillus oryzae*, etc. In this connection, it is said that many of the lactic acid bacteria that require this mevalonic acid produce bacteriocin.

Moreover, lactic acid bacteria are used as a countermeasure to prevent the propagation of harmful bacteria and also used to promote propagation of beneficial bacteria during the course of fermentation of miso and soy sauce. In addition, miso and soy sauce include 10% or more of sodium chloride with the exception of some sweet miso; accordingly, the lactic acid bacteria change to salt-resistant lactic acid bacteria during fermentation.

As seen from the above, products that use koji mold become the respective product via a process which involves symbiosis of koji mold, lactic acid bacteria and yeast; however, there are no brewed health foods, such as intestinal aids for humans and animals, etc. that use the effect of symbiosis of koji mold, lactic acid bacteria and yeast on a grain base.

Yogurt is an example of a health food that uses lactic acid bacteria, but yogurt is not a food that uses koji mold.

Many antibiotics have become popular since the clinical application of penicillin in 1941, and these antibiotics play an important role in infection. However, the frequent use of antibiotics has also lead to the appearance of drug-resistant bacteria such as MRSA (methicillin-resistant staphylococcus aureus) and VRSE (vancomycin-resistant staphylococcus epidermidis), etc. People and domestic animals are being administered the same antibiotics for infections common to people and domestic animals; as a result, drug-resistant bacteria are present in many hog and poultry farms, and this poses a serious social problem. At the present time there are no solutions to this problem.

A considerable amount of research has proceeded on the effects of symbiosis of microorganisms, with special emphasis on lactic acid bacteria; but yogurt, which is a milk product, appears to still be the only food product in which symbiosis of microorganisms is used. Apart from the above-described inhibiting activity of lactic acid bacteria on the propagation of putrefying bacteria and pathogenic bacteria under reduced pH by the production of short-chain fatty acids, lactic acid, etc., antibiotics such as bacteriocin, which is a peptide, etc. and mucilaginous polysaccharides are also receiving attention.

Moreover, the results of studies on biological activity, which is a potential strength of lactic acid bacteria, can be expected in the future.

Consequently, the appearance of resistant bacteria that was seen as a result of the administration of antibiotics will probably not pose a problem with the inhibition of the propagation of harmful bacteria (infectious disease pathogenic bacteria, etc.) by probiotics, and therefore, it appears that probiotics can be expected to be very important in the future.

Moreover, inhibition of propagation of *Salmonella* in the intestines of domestic animals by beneficial microorganisms, mainly lactic acid bacteria, has been presented (see PCT Application Japanese National Publication No. H9-506625). In this prior art, emphasis is placed on the fact that a symbiont of 29 species of bacteria of mainly lactic acid bacteria cultivated from the contents of the cecum of poultry produces a short-chain fatty acid aversive to *Salmonella* and a symbiont is presented that inhibits the propagation of *Salmonella* bacteria in the intestines of domestic animals. This prior art involved cultivation of a bacterial symbiont using cecal contents as the nutrients, and therefore, it was expected that this bacteria group would remain in the intestines to produce short-chain fatty acids and inhibit the propagation of *Salmonella* bacteria.

However, in this prior art, pre-cultivation is performed using the cecal contents of chickens in order to obtain intestinal activity of the bacteria; as a result the following inconvenience encounters: the cecal contents of chickens must be thoroughly considered from the point of hygiene, and very close sanitary controls must be implemented during the cultivation of the bacterial symbiont. Thus, this method is not practical and is inappropriate for food.

In view of the above, there is a demand for the development of a material which promotes the propagation of beneficial microorganisms and consist of nutrients that are superior in terms of hygiene, are easily produced and are inexpensive to produce, thus being different from nutrients that pose hygiene problems such as the cecal contents of chickens, etc.; and there is a further demand for the development of a technology which makes it possible to propagate beneficial microorganisms at the same time as the production of such a beneficial microorganism propagation-promoting material.

### Disclosure of the Invention

The present invention is based on these points and uses symbiosis of koji mold with yeast and lactic acid bacteria; and the object is to provide a material for the propagation of beneficial microorganisms that axe effective in maintaining the health of living beings during the rearing of living beings using a product whose starting material is grains as proposed by the present applicant in Japanese Patent Application Laid-Open (Kokai) No. H7-23725 and to provide a process for prepare the material for promoting the propagation of beneficial microorganisms.

Another object of the present invention is to provide a beneficial microorganism propagation-promoting material that is excellent in terms of hygiene, can be easily produced, and is inexpensive to produce, and also to provide a process in which the beneficial microorganisms can be propagated at the same time as the manufacture of such a beneficial microorganism propagation-promoting material.

In the above-described Japanese Patent Application Laid-Open (Kokai) No. H7-23725 filed by the applicant of the present invention, a product whose starting material is grains is proposed with which the phytic acid in the grains can be easily removed with the grains remaining in solid form, the activity of the vitamin B group, etc. contained in the product remains high, the minerals contained in such a product can be easily absorbed, this absorption can be promoted, and the product can be obtained at a low production cost; and a process for preparing such a product is also proposed therein.

The present inventors performed intense studies of such a product whose starting material is grains presented in the above-described application by the present applicant in order to accomplish the above-described object and completed the present invention upon discovering that by incorporating probiotics technology, a product obtained using these grains as the starting material can promote propagation of beneficial microorganisms that are effective in maintaining the health of living beings, and that koji mold and the beneficial microorganisms promote propagation of beneficial microorganisms when they are present as a symbiont with the product whose source is the grains.

In further detail, the probiotics used here is produced so that koji mold is inoculated on grains to effect koji preparation, the proteins and/or saccharides contained in the resultant from the koji preparation are hydrolyzed by adding water to such a resultant, and a predetermined amount of the phytic acid contained in the grains is decomposed and removed; and during this process, an environment is created in which beneficial microorganisms such as lactic acid bacteria and yeast or bifidobacteria, etc. can be symbiotic. In other words, the present invention uses the fact that the lactic acid bacteria and yeast or bifidobacterium, etc. can actively propagate while in the form of a symbiont with koji mold in the above-described product that forms the same base.

The inventors performed farther studies and discovered that the above-described beneficial microorganisms, including lactic acid bacteria, that actively propagate while in the form of a symbiont in the above-described product (1) can usually propagate within the intestines of humans and domestic animals, (2) do not have a detrimental effect on the host, such as humans and domestic animals, etc., and (3) can efficiently produce short-chain fatty acids, etc. within the intestines. Moreover, it is also discovered that the above-described product contains substances that become the nutrients of lactic acid bacteria, etc. growing in the intestines of domestic animals and remains undigested up to the colon, making it possible for the short-chain fatty acids aversive to harmful bacteria, including *Salmonella*, etc. to be produced (undigested product = enzyme undecomposed product); and thereupon the present invention was completed. In addition, it is further discovered that by using a combination of the above-described product and resistant starch (starch and partial hydrolysis product of starch that will not be digested and absorbed in the small intestines of healthy humans), the enzyme undecomposed product and resistant starch together become the nutrient of lactic acid bacteria., etc. that can grow in the intestines of domestic animals and thereby completed the present invention. Probiotics originally are living microorganisms. For instance, there are products that are a mixture of koji mold, yeast and lactic acid bacteria, etc.; however, as yet, there have been no products like that of the present invention, products of cultivation that contain fungal bodies produced from koji mold, yeast and lactic acid bacteria, etc. in the common base (grains).

As previously described, products (such as refined sake, miso, soy sauce, etc.) brewed using koji mold are obtained by methods in which koji mold, lactic acid bacteria and yeast are symbiotic, but these methods are effective in terms of product quality and are not for the purpose of probiotics. There are many cases where the live bacteria in the refined sake or soy sauce product are killed and live bacteria can become a problem in terms of quality control, even with miso. Moreover, since the initial stage and fermentation period is long with these brewed products, transition of these microorganisms may occur, and it is not possible to obtain a cultivated product of large quantities of koji mold, yeast. lactic acid bacteria, etc. in the same base (grains).

The beneficial microorganism propagation-promoting material of the present invention that is obtained in this way is characterized in that it is produced by inoculating koji mold on grains to effect koji preparation, adding water to a resultant from the koji preparation to thereby hydrolyze proteins and/or saccharides contained in the resultant, and removing a certain amount of phytic acid contained in the grains. This beneficial microorganism propagation-promoting material provides the beneficial microorganisms with nutrients in symbiosis with the koji mold when it is added to food containing these beneficial microorganisms and thereby promotes propagation of the beneficial microorganisms so that the health-promoting activities of the beneficial microorganisms are realized, while at the same time reliably inhibits propagation of harmful bacterial, thus improving maintenance of the health of the living being that consumes this food. In addition, this product includes a substance that becomes a nutrient of lactic acid bacteria, etc. that can grow in the intestines of humans and domestic animals and remains undigested by the time it reaches the colon so that the short-chain fatty acids (acetic acid, butyric acid, propionic acid, lactic acid, succinic acid, etc.), aversive to harmful bacteria, such as *Salmonella*, etc. will be produced (undigested substance = enzyme undecomposed substance); therefore, the beneficial microorganisms, such as lactic add bacteria. etc. can produce the short-chain fatty acids aversive to harmful bacteria, such as *Salmonella*, etc. using the undigested product in the intestines of animals as the nutrient and reliably inhibit propagation of *Salmonella*. Moreover, in contrast to the above-described conventional example, the cecal contents of chickens are not used as the nutrient, and therefore, the present invention is superior from the point of hygiene.

Furthermore, when resistant starch is added to the above-described product to obtain the beneficial microorganism propagation-promoting material, the enzyme undecomposed product contained in the above-described product and the resistant starch together form large quantities of nutrients for lactic acid bacteria, etc., that can grow in the intestines of domestic animals, and the beneficial microorganisms, such as lactic acid bacteria, etc. can grow more of the short-chain fatty acids aversive to harmful bacteria, such as *Salmonella*, etc. using the undigested product in the intestines of the animal as the nutrient, thus the propagation of the harmful bacteria, such as *Salmonella*, etc can be controlled more reliably.

Moreover, the beneficial microorganism propagation-promoting material of the present invention is characterized in that propagation of beneficial microorganisms contained in the resultant from the koji preparation and/or those added to such a resultant from the koji preparation is promoted during the hydrolysis. In this beneficial microorganism propagation-promoting material, the koji mold and beneficial microorganisms are symbiotic in the above-described resultant which has the same base, and the beneficial microorganisms receive nutrients from the resultant so that its propagation is promoted; thus, the koji mold and beneficial microorganism are cultivated together in such a resultant. Consequently, when this beneficial microorganism propagation-promoting material is added to food, propagation of the beneficial microorganisms that have already been cultivated in the beneficial microorganism propagation-promoting material and beneficial microorganisms contained in the food is further promoted so as to realize the health-promoting activities of the beneficial microorganisms and to reliably inhibit the propagation of harmful bacteria, such as *Salmonella*, thus improving the health maintenance of the living being that consumes the food. The Abeneficial microorganisms that have already been cultivated≅ refers to beneficial microorganisms that are not only naturally mixed during the preparation process but also added to the hydrolysis step, thus including lactic acid bacteria that require the mevalonic acid or produce bacteriocin, and others.

The process for preparing the beneficial microorganism propagation-promoting material of the present invention is characterized in that koji mold is inoculated on grains to effect koji preparation, water is added to a resultant from the koji preparation to thereby hydrolyze proteins and/or saccharides contained in such a resultant, and a predetermined amount of phytic acid is removed from the grains, thus producing a beneficial microorganism propagation-promoting material that promotes the propagation of beneficial microorganisms.

According to this process of the present invention for preparing a beneficial microorganism propagation-promoting material, koji mold is inoculated to the grains, which are the starting material, so as to effect koji preparation, thus propagating koji mold and removing the phytic acid from the grains; and in addition, proteins and/or saccharides contained in the resultant from koji preparation are hydrolyzed by adding water to such a resultant while at the same time a predetermined amount of phytic acid is removed. Accordingly, a predetermined amount of phytic acid can be easily and reliably removed in a short amount of time with the grains kept in a solid form. Promotion of the propagation of the beneficial microorganisms further leads to realization of the various health-promoting effects of the beneficial microorganisms, and reliable inhibition of the propagation of harmful bacteria, such as *Salmonella* is accomplished. Thus, a beneficial microorganism propagation-promoting material that helps to sustain the health of living beings can be produced. Moreover, the production process is simple, and production cost is low.

In addition, according to the process of the present invention for preparing a product whose source is grains, it is characterized that the propagation of beneficial microorganisms in the resultant from koji preparation and/or beneficial microorganisms added to the resultant from a koji product is promoted during the above-described hydrolysis process.

In the process of the present invention for preparing a beneficial microorganism propagation-promoting material, koji mold and beneficial microorganisms are symbiotic in the koji preparation resultant that has the same base during hydrolysis, and propagation of the beneficial microorganisms is promoted when the beneficial microorganisms receive nutrients from the resultant so that the koji mold and beneficial microorganisms are cultivated together in the resultant from the koji preparation. Accordingly, when this beneficial microorganism propagation-promoting material is added to food, propagation of the beneficial microorganisms that have been cultivated in the beneficial microorganism propagation-promoting material and of the beneficial microorganisms contained in the food is further promoted, so that the various health-promoting activities of the beneficial microorganisms are realized, and propagation of harmful bacteria, such as *Salmonella*, etc. can be reliably inhibited, thus improving the health maintenance of the living beings that consumes such a food. In addition, the process for preparing the beneficial microorganism propagation-promoting material that acts in this way is simple, and it can be produced at a low cost.

As to the above-described beneficial microorganism, at least one of Eumycetes, lactic acid bacteria and bifidobacteria can be selected, and they can be selected as needed in accordance with the use of the beneficial microorganism propagation-promoting material.

The beneficial microorganism propagation-promoting material and the process for preparing the same in accordance with the present invention are formed and function as described above. Accordingly, the product whose starting material is grains that has been proposed by the present applicant can be used, and it is possible to retain the effect of the product obtained from the grains as the starting material and promote propagation of beneficial microorganisms that are effective in maintaining the health of living beings.

More specifically, with the use of the product whose source is grains, the phytic acid in the grains can be easily removed with the grains kept in a solid form. Minerals, etc. contained in the product can be easily absorbed by way of keeping the high activity of the vitamin B group, etc. contained in the product, and a product that can further promote such an absorption can be obtained. Moreover, the preparation process is simple, and the production cost is low.

Furthermore, the product contains substances that become the nutrients of lactic acid bacteria, etc. growing in the intestines of domestic animals and remains undigested up to the colon, making it possible for the short-chain fatty acids aversive to harmful bacteria, including *Salmonella*, etc. to be produced (undigested product = enzyme undecomposed product); accordingly, the beneficial microorganisms, such as lactic acid, etc. can produce short-chain fatty acids aversive to harmful - bacteria, including *Salmonella*, using the undigested product in the intestines of the animal as the nutrients and reliably inhibit propagation of harmful bacteria, such as *Salmonella*. Moreover, in contrast to the above-described prior art, the contents of the cecum of chickens are not the nutrients; therefore, the present invention is by far superior in terms of hygiene.

Furthermore, when a resistant starch is added to the above-described product to make a beneficial microorganism propagation-promoting material, the enzyme undecomposed product contained in the above-described product and the resistant starch together become large amounts of nutrient for lactic acid bacteria, etc. that grow in the intestines of humans and domestic animals; and more short-chain fatty acids aversive to harmful bacteria, such as *Salmonella*, etc., are produced by the beneficial microorganisms, such as lactic acid bacteria, etc. with the undigested product in the intestines of the animals serving as the nutrients, thus more reliably controlling the propagation of harmful bacteria, such as *Salmonella*, etc.

Furthermore, when the beneficial microorganism propagation-promoting material is added to, for instance, food containing beneficial microorganisms, propagation of such microorganisms is promoted by the beneficial microorganism propagation-promoting material, the various health promoting activities of the beneficial microorganisms can be realized, and propagation of the harmful bacteria, such as *Salmonella*, etc., can be inhibited, so that the health of living beings that consume such a food is improved. Thus, propagation of beneficial microorganisms that are effective in maintaining the health of a living being during its rearing can be promoted.

Moreover, when the beneficial microorganisms are introduced by a natural cause or by an intentional addition during hydrolysis of the resultant from koji preparation, koji mold and beneficial microorganisms are symbiotic during hydrolysis in such a resultant that has the same base, and propagation of the beneficial microorganisms is promoted when the beneficial microorganisms receive nutrients from the resultant, so that the koji mold and beneficial microorganisms are cultivated together in the resultant from the koji preparation. Thus, when this beneficial microorganism propagation-promoting material is added to food, propagation of the beneficial microorganisms that have been cultivated in the beneficial microorganism propagation-promoting material and of the beneficial microorganisms contained in the food is further promoted, so that the various health-promoting activities of the beneficial microorganisms are realized, and propagation of harmful bacteria, such as *Salmonella*, etc. can be reliably inhibited, thus improving the health maintenance of the living beings that consume such a food.

Furthermore, according to the present invention, the beneficial microorganism propagation-promoting material described above are prepared by a simple process at a low cost.

### Brief Description of the Drawings

Figure 1 is a characteristic diagram showing changes in microorganisms in Ayamahai-moto≅;
Figure 2 is a diagram according to one embodiment of the process of the present invention for preparing product from which phytic acid has been removed from defatted soybeans;
Figure 3 is a characteristic diagram showing the effect of administering fermented soybean feed and untreated soybean feed on cecal content pH and cecal total fatty acid amount; and
Figure 4 is a characteristic diagram showing the effect of fermented soybean feed and untreated soybean feed on cecal contents.

### Best Mode to Carry Out the Invention

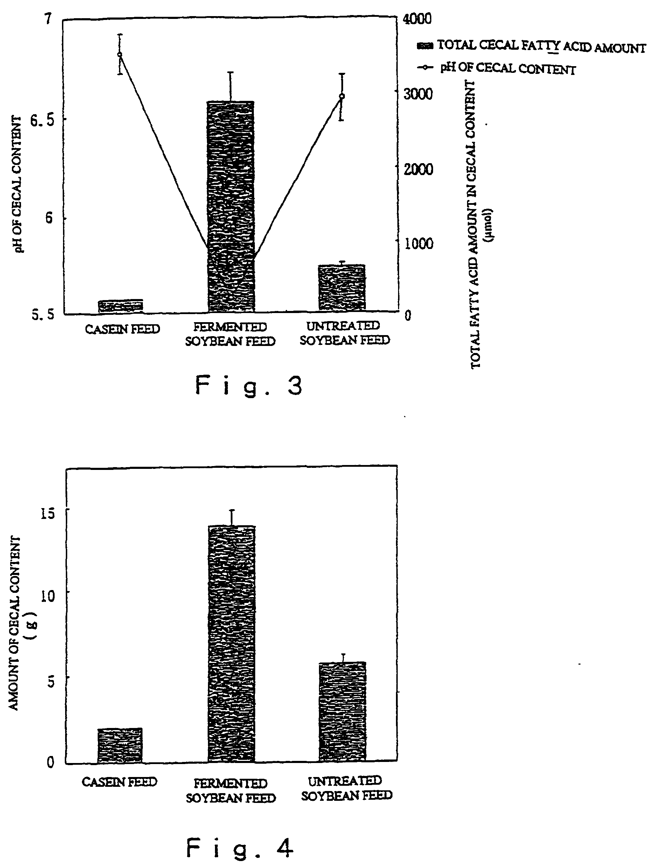

Embodiments of the present invention will be described with reference to Figures 2 through 4.

Figure 2 illustrates one embodiment of the process for preparing the beneficial microorganism propagation-promoting material from defatted soybeans, a type of grain, according to the present invention which is represented by the solid lines; and another embodiment of the process of the present invention is represented by the dotted lines in the same figure.

First, an embodiment of the process of the present invention will be described with reference to the processes shown by the solid lines in Figure 2.

Defatted soybeans are first cooked. By effecting the cooking, propagation of koji mold is promoted further compared to the case where cooking is not performed. Cooking of the defatted soybeans can be performed by the batch method whereby cooking and then koji preparation are performed separately or by the continuous method whereby a device for preparing koji mold is used so that cooking and koji preparation are done continuously, depending on the purpose of production, etc.

After completion of the cooking, the defatted soybeans are cooled to adjust the water content in the defatted soybeans to a level allowing koji mold to propagate (for example, 36% by weight).

The process of the present invention is performed on a defatted soybean with its water content adjusted as described above.

More specifically, when the defatted soybeans that have been cooked are cooled to 40°C or lower, the defatted soybeans are inoculated with a koji starter comprising koji mold a predetermined weight ratio, and mixing is conducted to uniformness.

Then, the mixture is set aside in a device for preparing koji while keeping product temperature at approximately 32°C; as a result, the defatted soybeans are fermented by the koji mold, that is, koji preparation is promoted, and the product is wrung as temperature increases to approximately 38°C. Then rotary agitation of the product that promotes production of koji mold is performed as the first agitation treatment so that the product is unwrapped, and air is supplied to the inside for aeration so that the product temperature is brought down to approximately 33 to 35°C. Aeration is continued at this temperature so as to promote koji preparation treatment. A second agitation is performed, and aeration is continued. The defatted soybeans are thus fermented by the koji mold, and koji preparation is continued until the phytic acid in the defatted soybeans are reduced by a predetermined amount.

In this case, the enzymes that decompose phytic acid, or phytase and phosphatase, which are produced by the koji mold as a result of koji mold propagation in the defatted soybeans, decompose and eliminate the phytic acid contained in the defatted soybeans.

In other words, enzymes that decompose phytic acid decompose a predetermined amount of phytic acid from the phytic acid, which is a compound where phosphoric acid groups are bonded to all of the hydroxyl groups of myoinocitol, so that the phosphoric acid groups are liberated and a culture that promotes propagation of beneficial microorganisms, such as lactic acid bacteria, etc., is formed as the vitamin B group of myoinocitol.

The koji mold used in this koji preparation is koji mold that has long been used in Japanese fermented food products and tempe; for example, koji mold from the genus *Aspergillus* and the genus *Rhizopus* that are safe for food, such as *Aspergillus usami*, *Aspergillus kawati*, *Aspergillus awamori*, *Aspergillus saitoi, Aspergillus oryzae, Aspergillus niger*, etc. are desirable for use.

In regard to the fermentation time, the fermentation time is set at least 24 hours or longer in accordance with the type of koji mold used. It is desirable that the fermentation time is sufficient to eliminate a predetermined amount of phytic acid from the defatted soybeans.

In this embodiment, phytic acid is further removed and also hydrolysis of proteins and/or saccharides is performed. The term saccharides here includes saccharides, such as oligosaceharides, etc. and starch, etc. Hydrolysis after koji preparation can also be performed with the base still in a flake form.

More specifically, in this embodiment, once production of the koji is completed, water is added to the product to a water content of, for instance, 50% by weight; and then the product is heated to 30 to 45°C and kept at this temperature for a predetermined amount of time, for instance, the time needed to bring the number of lactic acid bacteria which are beneficial microorganisms to 10⁸ cfu/g or more, so as to obtain the product.

Moreover, removal of the phytic acid is performed by liberating at least 1 phosphoric acid group from the phytic acids consisting of inocitol 6 phosphoric acid, butinocitol 4 phosphoric acid, inocitol 3 phosphoric acid, inocitol 2 phosphoric acid, inocitol 1 phosphoric acid, and inocitol from which at least 2 phosphoric acid groups have been liberated are water soluble and have the effect of strongly promoting absorption of minerals consisting of calcium contained in products whose starting material is grains.

In further detail, the above-described inocitol 6 phosphoric acid and inocitol 5 phosphoric acid have strong ionic bonds and do not elute the bonded calcium so that calcium-absorbing effects are strongly inhibited. In contrast to this, inocitol 4 phosphoric acid to inocitol 1 phosphoric acid bond well with calcium and have optimal affinity for easily eluting the bonded calcium when necessary, and therefore, the above-described characteristic activity of promoting calcium absorption is realized. In this case, the number of phosphoric acid groups liberated from the phytic acid can be controlled by adjusting the fermentation time and hydrolysis time, as well as hydrolysis temperature, in accordance with: the type, state, properties and molecular weight of the grain; the type, state, properties and molecular weight of the koji mold; and the type, properties, etc. of the product.

When added to food containing beneficial microorganisms, the beneficial microorganism propagation-promoting material of the present embodiment prepared as described above provides nutrients to the beneficial microorganisms, forms a symbiont with koji mold, promotes propagation of the beneficial microorganisms, and realizes the various health-promoting activities of the beneficial microorganisms; in addition, it reliably can control propagation of harmful bacteria. As a result, it is possible to improve the health maintenance of the living beings that consume such a food.

Furthermore, this product contains a substance that becomes the nutrient of lactic acid bacteria, etc., which can easily grow in the intestines of domestic animals, and remains undigested up to the colon, where the short-chain fatty acids aversive to harmful bacteria, including *Salmonella*, etc., can be produced (undigested product = enzyme undecomposed product). Accordingly, the beneficial microorganisms, such as lactic acid, etc. produce short chain fatty acids aversive to harmful bacteria, including *Salmonella*, by using the undigested product in the intestines of the animal as the nutrient and reliably inhibit propagation of harmful bacteria, such as *Salmonella*, etc. Moreover, unlike the prior art described above, the contents of the cecum of chickens are not the nutrients and therefore, the present invention is by far superior in terms of hygiene.

By adding resistant starch to the above-described product to obtain a beneficial microorganism propagation-promoting material, the enzyme undecomposed product contained in the product and the resistant starch together form nutrients for lactic acid bacteria, etc. that can grown in the intestines of domestic animals; and the beneficial microorganisms, such as lactic acid, etc. produce short chain fatty acids aversive to harmful bacteria, including *Salmonella*, by using the undigested product in the intestines of the animal as the nutrients and reliably inhibit propagation of harmful bacteria, such as *Salmonella*, etc.

Next, another embodiment of the process of the present invention will be described with reference to where it is shown by the dotted line in Figure 2.

In this embodiment, propagation is promoted with koji mold and beneficial microorganism symbiotic during the hydrolysis process of a product obtained by koji preparation.

Consequently, the same process as in the above-described embodiment is performed until koji preparation is completed; but in this embodiment, beneficial microorganism is introduced to the product by adding the beneficial microorganism to the product of koji preparation at the time of hydrolysis and/or by using the beneficial microorganism that is naturally contained in the grains that serve as the starting material.

The beneficial microorganism is a Eumycetes, such as yeast, etc., lactic acid bacteria (including lactic acid bacteria that require Ahiochii≅ acid (mevalonic acid)), bifidobacterium, and other beneficial bacteria, and it include all bacterium that is effective in maintaining the health of living beings when living beings are being reared. The Eumycetes, lactic acid bacteria and bifidobacteria are very compatible with the grain that serves as the base and propagate using the grain as their nutrient. Moreover, at least one type of beneficial microorganism can be introduced to the product of koji preparation in accordance with the type of grain that serves as the base, the type of food, its use, etc., as the final product that is to be produced.

When hydrolysis is started by introducing beneficial microorganism to the product of koji preparation, the koji mold that have propagated during koji preparation and the beneficial microorganisms form a symbiont in the product with the same base during hydrolysis; as a result, the beneficial microorganisms receive nutrients from the product and their propagation is promoted, so that the koji mold and beneficial microorganisms are cultivated together in the product. In particular, the vitamin B group is produced during hydrolysis in the product obtained by koji preparation, and components with a high nutritional value that can be easily absorbed by the beneficial microorganisms are produced; accordingly, propagation of the beneficial microorganisms is promoted with these components serving as the nutrients. Furthermore, the various components that have been hydrolyzed by the enzymes of koji mold are the equivalent of the defatted soybean decomposed by digestive enzymes, and they are separated into digested product and undigested product in the product of the present embodiment. Although the decomposed product (digested product) of hydrolysis by the enzymes of koji mold is directly absorbed when consumed by humans and domestic animals, the undecomposed product becomes, without being absorbed, a nutrient of beneficial lactic acid bacteria that are consistently present in the intestines, so that production of short-chain fatty acids that are beneficial to humans and domestic animals is increased.

Moreover, the above-described product is a medium in which lactic acid bacteria can actively propagate; accordingly, the lactic acid bacteria, etc. that has propagated can usually exist in the intestines of domestic animals with the enzyme undecomposed product contained in the product consumed as a nutrient. As a result, beneficial microorganism propagation-promoting material that uses probiotics where koji mold and beneficial microorganisms are cultivated in a product with the same base is produced in the end.

When the beneficial microorganism propagation-promoting material of the present embodiment thus produced is added to food, propagation of the beneficial microorganisms that have already been cultivated in the beneficial microorganism propagation-promoting material and of the beneficial microorganisms contained in the food is further promoted; as a result, the various health-promoting effects of the beneficial microorganisms can be realized and propagation of harmful bacteria, such as *Salmonella*, etc. can be reliably prevented to prevent infection and improve the health maintenance of the living beings consuming the food. In particular, when lactic acid bacteria are inoculated *in vivo* in humans or animals, whether as live or dead bacteria, their biological activity is activated *in vivo*, and very marked health-promoting activity is realized.

The beneficial microorganism propagation-promoting material of the present invention will now be described with actual examples.

Table 1 shows the content of vitamin B groups and isoflavone compounds in a fermented defatted soybean Aa produced by the process of the present invention using *Aspergillus awamori* as the koji mold and untreated defatted soybean.

**Table 1**

| | Soybean scraps Aa | Untreated soybean scraps |
|---|---|---|
| Thiamin (vitamin B1) | 0.40 (mg/100g) | 0.61 (mg/100g) |
| Riboflavin (vitamin B2) | 0.67 (mg/100g) | 0.31 (mg/100g) |
| Vitamin B6 | 0.88 (mg/100g) | 0.67 (mg/100g) |
| Folic acid | 0.34 (mg/100g) | 0.33 (mg/100g) |
| Pantothenic acid | 3.64 (mg/100g) | 1.88 (mg/100g) |
| Biotin | 63.7 (µg/100g) | 46.9 (µg/100g) |
| Niacin | 2.48 (mg/100g) | 2.00 (mg/100g) |
| Free inocitol | 450 (mg/100g) | 36 (mg/100g) |
| Daidzin | 5.4 (mg/100g) | 120 (mg/100g) |
| Daidzein | 80 (mg/100g) | 3.7 (mg/100g) |
| Genistin | 13 (mg/100g) | 170 (mg/100g) |
| Genistein | 78 (mg/100g) | 4.1 (mg/100g) |

It is clear from Table 1 that the vitamin B group content, including riboflavin, vitamin B6, pantothenic acid, biotin, free inocitol, etc. is greatly increased with the fermented defatted soybean treated with shochu koji mold produced by the present invention when compared to untreated defatted soybean. In particular, the amount of free inocitol is much higher than with untreated defatted soybean because the koji mold have been broken down by a phytic acid-decomposing enzyme. Consequently, it is clear that the beneficial microorganism propagation-promoting material of the present invention has a high vitamin B group content, which is a nutrient with high nutritional value that is very easily absorbed by beneficial microorganisms, for realizing probiotics. Thus, it is clear that the beneficial microorganism propagation-promoting material of the present invention heightens the symbiosis of koji mold and beneficial microorganisms with makes probiotics very possible.

Moreover, according to Table 1, isoflavone compounds are as easily decomposed as phytic acid. That is, although the amounts of daidzin and genistin, which are glycosides, are very high in comparison to the aglycons daidzein and genistein with the untreated defatted soybean, the daidzin and genistin glycosides are broken down so that very little remains in the fermented defatted soybean Aa produced by the present invention, while the amounts of daidzein and genistein, which are aglycons, produced by decomposition are quite high.

Thus, according to the present invention, of the isoflavone compounds in defatted soybean, it is possible to produce aglycons with strong pharmaceutical activity at a very high production ratio. The beneficial microorganism propagation-promoting activity of the beneficial microorganism propagation-promoting material of the present invention with the above-described properties will be explained with the results of property identification tests.

### Test 1

Test of promotion of lactic acid bacteria fermentation by defatted soybean, etc. that fermented two types of koji mold by the present invention.

### Object

The object is to investigate promotion of lactic acid bacteria propagation by the addition of a defatted soybean, etc. that fermented two types of koji mold to cow's milk as the sample.

### Test method

One-hundred milliliters of cow's milk were introduced to five beakers. Only the cow's milk was introduced as the Control group, 0.6% untreated soybean and oligosaccharide were added for Comparative Groups 1 and 2, respectively, 0.6% fermented defatted soybean Aa that used *Aspergillus awamori* was added for Test Group 1, and 0.6% fermented defatted soybean (AO) that had been produce under the same conditions as fermented defatted soybean Aa using *Aspergillus oryzae*, which is a miso koji mold, as the koji mold, was added for Test Group 2. Then each of the beakers was kept at 40°C over a water bath, pH was determined, and the milk was checked for odor after 0, 1, 4, and 20 hours.

### Results

pH and odor of the milk changed as shown in Table 2.

**Table 2**

| | Control Group | Comparative Group 1 | Comparative Group 2 | Experimental Group 1 | Experimental Group 2 |
|---|---|---|---|---|---|
| Additive | None | Untreated soybean | Oligosaccharide | Soybean scrap Aa | soybean scrap AO |
| After 0 hours | 6.65 | 6.67 | 6.66 | 6.56 | 6.58 |
| After 1 hour | 6.56 | 6.61 | 6.58 | 6.46 | 6.48 |
| After 4 hours | 6.57 | 6.66 | 6.58 | 6.45 | 6.44 |
| After 20 hours | 6.30 | 6.20 | 6.52 | 6.23 | 4.88 |
| Smell | Milk odor | Putrefying odor (+++) | Milk odor | Putrefying odor (+) | Yogurt odor (+++) |
| The strength of the smell is shown in parentheses: (+) : slight odor (++) : considerable odor (+++) : strong odor | | | | | |

Based on Table 2, it can be said that there were no changes in smell and lactic acid bacteria did not propagate in the Control Group with milk only or in Comparative Group 2 where oligosaccharide was added. It can be said that a strong putrefying odor (putrefying odor of fish = amine odor) was obtained by Comparative Group 1 to which untreated soybean had been added and that there was no propagation of lactic acid bacteria.

In contrast to this, although faint, there was a putrefying odor given off in Test Group 1 where fermented defatted soybean Aa was added, but this odor was not an amine odor. This fact indicates that lactic acid bacteria, which were naturally occurring in fermented defatted soybean Aa, propagates and the lactic acid bacteria that has propagated controls to a certain extent the activity of putrefying bacteria in the cow's milk, which does not originally contain lactic acid bacteria, so that extreme putrefaction of the cow's milk is prevented.

Moreover, pH was much lower when compared to the other groups in Test Group 2 to which fermented defatted soybean (AO) produced by the process of the present invention using *Aspergillus oryzae*, which is a miso koji mold, had been added; and there was no putrefying odor, but rather a strong yogurt odor. This fact shows that when defatted soybean (AO) that has been fermented using *Aspergillus oryzae*, which is a miso koji mold, are added, the beneficial microorganism propagation-promoting material of the present invention is superior for the efficient propagation of lactic acid bacteria that are naturally occurring in the soybeans.

Based on the results from Test Groups 1 and 2, it is clear that propagation of lactic acid bacteria, which are beneficial microorganisms that were added to the product of koji preparation during the hydrolysis process of the present invention, is promoted. In other words, it is clear that the fermented defatted soybean Aa and fermented defatted soybean (AO) of the present invention contain a lactic acid bacteria growth-promoting factor and that growth of other harmful bacteria is inhibited by propagation of the lactic acid bacteria. It is clear that this effect is particularly strong with fermented defatted soybean (AO) that was produced with *Aspergillus oryzae* and that a strong probiotic action takes place. When explained further, it is clear that the koji mold and beneficial microorganisms form a symbiont in the above-described product with the same base during hydrolysis and the beneficial microorganisms receive nutrients from the product so that propagation is promoted and the koji mold and beneficial microorganism are cultivated together in the product. Consequently, when this beneficial microorganism propagation-promoting material is added to food, propagation of the beneficial microorganisms that have already been cultivated in such a beneficial microorganism propagation-promoting material and beneficial microorganisms contained in the food is further promoted to realize health-promoting activities of the beneficial microorganisms and to reliably inhibit the propagation of harmful bacteria, thus improving the health maintenance of the living beings that consume the food.

### Test 2

Test on promotion of lactic acid bacteria propagation by defatted soybeans fermented by *Aspergillus oryzae* (AO), a miso koji mold, of the present invention.

### Object

The object of this test is to investigate the promotion of lactic acid bacteria propagation by adding fermented defatted soybean (AO) that was confirmed to have lactic acid bacteria propagation-promoting activity by Test 1 to a sample containing lactic acid bacteria.

### Test Method

One-hundred milliliters of cow's milk and 20 ml commercial lactic acid bacteria drink (brand name: AYakurt≅) were introduced to four individual beakers, and fermented soybean (AO) was omitted from the Comparative Group, while 0.5%, 1.0% and 2.0% fermented defatted soybean (AO) was added to Test Groups 1, 2 and 3, respectively. Then each beaker was kept at 40°C over a water bath and checked for solidification of the cow's milk and pH of the cow's milk for 22 hours.

### Results

### (1) Solidification of cow's milk

Solidification of the cow's milk was seen in all of Test Groups 1, 2 and 3, where 0.5%, 1.0% and 2.0% fermented defatted soybean (AO) had been added, but solidification of cow's milk was not found in the Comparative Group. Solidificaton started after four hours, after two hours, and after one hour in each of Test Groups 1, 2 and 3, respectively. Since there was almost no change in initial pH after 4 hours, the solidification of cow's milk that occurred when fermented defatted soybean (AO) had been added was probably not due to lactic acid bacteria, but rather to the protein-decomposing enzyme contained in the fermented defatted soybean (AO).

### (2) pH of cow's milk

pH of the cow's milk changed as in Table 3.

**Table 3**

| | Comparative Group | Test Group 1 | Test Group 2 | Test Group 3 |
|---|---|---|---|---|
| Amount of defatted soybean (AO) added | Nothing added | 0.5% added | 1.0% added | 2.0% added |
| Solidification of cow's milk | No solidification | Solidified after 4 hours | Solidified after 2 hours | Solidified after 1 hour |
| pH (initial) | 6.04 | 6.00 | 5.94 | 5.85 |
| pH (after 4 hours) | 5.95 | 5.92 | 5.88 | 5.81 |
| pH (after 22 hours) | 5.36 | 3.88 | 3.82 | 3.84 |

Although significant changes were not seen overall in terms of pH after four hours, Table 3 does show that in contrast to the pH of 5.36 in the Comparative Group, it was approximately pH of 3.8 or higher in each of Test Group 1, 2 and 3 where 0.5%, 1.0% and 2.0% fermented defatted soybean (AO) had been added after 22 hours. This means that lactic acid bacteria do propagate, and pH of the cow's milk does drop when fermented defatted soybean (AO) is added. Moreover, the fact that there was an obvious reduction in pH of each of Test Group 1, 2 and 3 when compared to the Comparative Group after 22 hours means that when fermented defatted soybean (AO) of the present invention are added, the lactic acid bacteria efficiently propagate, and their growth is clearly abundant.

### Test 3

Test on the promotion of lactic acid bacteria propagation by fermented defatted soybean (AO) of the present invention in the presence of an antibiotic.

### Object

The object is to investigate the promotion of lactic acid bacteria propagation by adding fermented defatted soybean (AO) to a sample containing antibiotic and lactic acid bacteria.

### Test Method

One-hundred milliliters of cow's milk and 20 ml commercial lactic acid bacteria drink (brand name: AYakurt≅) were added to each of three beakers. Fermented defatted soybean (AO) was not added to the Comparative Group, while 0.5 and 1.0% fermented defatted soybean (AO) was added to Test Groups 1 and 2, respectively. No penicillin, 0.02 U/ml penicillin, and 0.2 U/ml penicillin were added to Comparative Group and Test Groups 1 and 2, respectively.

Then each beaker was kept at 40°C with a water bath, and pH of the cow's milk after 0, 1, 4 and 22 hours were measured.

### Results

pH of the cow's milk changed as in Tables 4, 5 and 6.

**Table 4**

| Comparative Group | No defatted soybean (AO) added | | |
|---|---|---|---|
| Penicillin | 0 U/mL | 0.02 U/mL | 0.2 U/mL |
| After 0 hours | 6.00 | 6.01 | 6.02 |
| After 1 hour | 5.94 | 5.94 | 5.95 |
| After 4 hours | 5.92 | 5.94 | 5.95 |
| After 22 hours | 5.05 | 5.15 | 5.77 |

**Table 5**

| Test Group 1 | 0.5% defatted soybean (AO) added | | |
|---|---|---|---|
| Penicillin | 0 U/mL | 0.02 U/mL | 0.2 U/mL |
| After 0 hours | 5.97 | 5.96 | 5.96 |
| After 1 hour | 5.91 | 5.91 | 5.92 |
| After 4 hours | 5.91 | 5.91 | 5.92 |
| After 22 hours | 4.09 | 4.16 | 4.56 |

**Table 6**

| Test Group 2 | 1.0% defatted soybean (AO) added | | |
|---|---|---|---|
| Penicillin | 0 U/mL | 0.02 U/mL | 0.2 U/mL |
| After 0 hours | 5.92 | 5.90 | 5.89 |
| After 1 hour | 5.86 | 5.85 | 5.83 |
| After 4 hours | 5.86 | 5.85 | 5.84 |
| After 22 hours | 4.05 | 4.13 | 4.37 |

According to Table 4, although there was almost no inhibition of growth in the Comparative Group when 0.02 U/mL penicillin was added, growth of the lactic acid bacteria was inhibited by the penicillin when 0.2 U/mL were added.

As seen from Tables 5 and 6, even though penicillin was added to the culture medium to which fermented defatted soybean (AO) had been added, lactic acid bacteria *(Lactobacillus casei)* did grow, and growth when 0.5% fermented soybean (AO) had been added to the culture medium treated with 0.2 U/mL penicillin was better than in the Comparative Group to which penicillin had not been added.

It is clear from above-described Tests 2 and 3 that when the beneficial microorganism propagation-promoting material of the present invention is added to food containing beneficial microorganisms, the beneficial microorganism are provided with nutrients and form a symbiont with the koji mold to promote propagation of the beneficial microorganisms and realize the various health-promoting activities of the beneficial microorganisms, as well as reliably inhibit the propagation of harmful bacteria and improve the health maintenance of living beings that consume the food. Thus, propagation of beneficial microorganisms that are effective in maintaining the health of a living being during its rearing can be promoted.

### Test 4

### Identification of lactic acid bacteria

### Object

The object is to propagate lactic acid bacteria that can survive in the intestines of humans and animals using intestinal contents as the nutrients and confirm whether or not the propagation of these lactic acid bacteria is promoted by fermented defatted soybean.

### Test method

Lactic acid bacteria were separated from fermented defatted soybean (AO) produced in accordance with the process of the present invention using *Aspergillus oryzae* of the koji mold for miso, and the test items in Table 7 were used to identify the lactic acid bacteria.

**Table 7**

| Test item | Test result |
|---|---|
| Morphology | Cocci |
| Gram staining | + |
| Spores | - |
| Mobility | - |
| Response to oxygen | Facultative anaerobic |
| Catalase | - |
| Production of gas from glucose | - |
| Lactic acid produced | L (+) |
| Growth at 10°C | + |
| Growth at 45°C | + |
| Growth in the presence of 6.5% NaCl | + |
| Growth at pH of 9.6 | + |
| GC content of DNA in fungal body mol%) | 40 |
| Note: G = guanine, C = cytosine | |

The lactic acid bacteria that had separated was identified as *Enterococcus* sp. based on the properties in Table 7. It was confirmed that this lactic acid bacteria is a bacteria normally present in the digestive tract called *Enterococcus*.

### Test 5

Study of inhibition of propagation of *Salmonella* by fermented defatted soybean (AO).

### Object

The object is to confirm the fact that the genus *Enterococcus*, which is an intestinal lactic acid bacteria, propagates in fermented defatted soybean (AO) produced by the process of the present invention using *Aspergillus oryzae*. Consequently, the following tests were performed on whether or not this fermented soybean (AO) has propagation-inhibiting activity on *Salmonella*.

### Test method

(1) Fermented, defatted soybean (product of the present invention) group
(2) Group where 2% lactose had been added to fermented defatted soybeans
(3) Group where 2% lactose and three types of lactic acid bacteria had been added to fermented defatted soybeans
(4) Defatted soybean group (Control group)

The water content of the above-described four groups was pre-adjusted to 50% by weight. Then two species of *Salmonella* (*Salmonella typhimurium* and *Salmonella enteritidis*) were inoculated into each and cultivated aerobically at 42°C. Samples were taken immediately after inoculation, 12 hours later and 24 hours later and *Salmonella* was determined.

### Results

The number of above-described *Salmonella* in each group changed as shown in Tables 8 and 9.

**Table 8**

| Changes in number of Salmonella typhimurium in each group | | | |
|---|---|---|---|
| Culture medium | Number of live Salmonella typhimurium (CFU/ml) | | |
| | 0 hours | 12 hours | 24 hours |
| Fermented soybean group (product of this invention) | 5.4 × 10³ | 2.1 × 10³ | < 10¹ |
| Fermented soybeans (lactose) | 5.7 × 10³ | 2.2 × 10³ | < 10¹ |
| Fermented soybeans (lactose, lactic acid bacteria added) | 5.5 × 10³ | 2.1 × 10³ | < 10¹ |
| Defatted soybean group | 2.1 × 10⁴ | 2.4 × 10⁵ | 3.1 × 10⁶ |

**Table 9**

| Changes in number of Salmonella enteritidis in each group | | | |
|---|---|---|---|
| Culture medium | Number of live Salmonella enteritidis (CFU/ml) | | |
| | 0 hours | 12 hours | 24 hours |
| Fermented soybean group (product of this invention) | 5.5 × 10⁴ | 3.4 × 10³ | < 10¹ |
| Fermented soybeans (lactose) | 5.7 × 10⁴ | 3.3 × 10³ | < 10¹ |
| Fermented soybeans (lactose, lactic acid bacteria added) | 3.4 × 10⁴ | 2.4 × 10³ | < 10¹ |
| Defatted soybean group | 3.6 × 10⁴ | 2.4 × 10⁵ | 4.1 × 10⁶ |

It was confirmed that when *Salmonella* was added to defatted soybeans, there was a tendency toward an increase over time, but there was a reduction over time in the fermented, defatted soybean group, with the number of bacteria being ten or lower and almost all of the *Salmonella* being killed in 24 hours. This is because since there was an increase in lactic acid bacteria with the fermentation of defatted soybeans by koji mold, as in the present invention, short-chain fatty acids, etc. were produced and the effects of inhibiting *Salmonella*, which are a harmful microorganism, were realized. There was not an increase in activity when lactose was added, and there was not an increase in activity when many lactic acid bacteria were added. This indicates that even if no special treatment is given, the inhibiting effects on harmful microorganism propagation of increasing lactic acid bacteria during the process of fermented soybeans is sufficient.

Furthermore, these findings confirm that the fermented defatted soybeans increase lactic acid bacteria when the fermented defatted soybeans are administered to animals, and therefore, inhibition of intestinal *Salmonella* propagation by lactic acid bacteria of fermented defatted soybeans can be expected, even if there is *Salmonella* infection, by administration of actual fermented defatted soybeans to humans and animals.

On the other hand, Imai et al. (Yushi, VII 1 A 43, No. 9, 62-70 (1990)) reports that there was an increase in *Salmonella* when *Salmonella* (*Salmonella enteritidis*) is inoculated into a mixture of half the volume raw eggs and a trace of karashi and soy sauce added to fermented soybeans and kept at 20°C. This shows that fermented soybeans are not inhibiting in terms of *Salmonella* growth.

### Test 6

Test on administration of fermented soybeans to animals (1)

### Object

The object of this test is to confirm the effect of adding fermented defatted soybean (AO) used in Test 5 on intestinal flora in weaned piglets.

### Test method

The test involved heat stress (30°C, relative humidity of 70 to 80% RH) loading of piglets immediately after weaning (three weeks old) and providing feed of fermented defatted soybean (AO) to which commercial artificial milk had been added for four weeks up to seven weeks post partum (0.1%, 0.5% by weight, or none added to feed). Fresh feces were collected for a total of three times, immediately after the study was started, five weeks post partum; and seven weeks post partum, and intestinal bacteria were studied in accordance with the method of Mitsuoka et al.

### Results

An increase in lactic acid bacteria present in fermented soybeans was seen among both the 0.1% and 0.5% groups at five weeks post partum (second week after starting administration). Many of the lactic acid bacteria were short rods that were not found in the untreated group. Moreover, changes were reported on Day 4 after starting administration with regard to feces properties; and although the feces were loose stools in the untreated group, they were normal among the treated groups.

The above-described findings confirm that the lactic acid bacteria propagated by fermented defatted soybean (AO) is the bacteria normally found in the digestive trace and that there is even an increase in the digestive tract of animals. Moreover, it was confirmed that the fermented defatted soybeans have inhibiting effects on diarrhea induced by stress.

### Test 7

### Administration of fermented soybeans to animals (2)

### Object

The object is to confirm the effects of administering fermented soybean Aa to rats on intestinal flora.

### Test method

Jcl:SD male rats at five weeks of age and weighing 100 to 120 g were reared for seven days on commercial solid feed and acclimated to their rearing environment. They were then divided into the three groups: casein feed (n = 7); fermented, defatted soybean feed (using *Aspergillus awamori* (n = 7)) (product of the present invention); and untreated defatted soybean feed (n = 5), and they were reared for three weeks. The composition of the feeds is shown in Table 10 below. The animals were given test feed and drinking water *ad libitum*. The properties of the cecal contents of the animals were compared after three weeks.

**Table 10**

| Component | Casein feed | Fermented, defatted soybean feed | Untreated, defatted soybean feed |
|---|---|---|---|
| Casein | 200 | - | - |
| Fermented, defatted soybean | - | 500 | - |
| Untreated, defatted soybean | - | - | 548 |
| Mineral mixture | 35 | 35 | 35 |
| Vitamin mixture | 10 | 10 | 10 |
| Corn oil | 50 | 50 | 50 |
| Soybean fiber | 25 | - | 13 |
| Sucrose | 200 | 200 | 200 |
| □ Corn starch | 630 | 405 | 344 |

### Results

The results were as shown in Figure 5 and Figure 6.

According to Figure 5, pH of the cecal contents of the group administered fermented, defatted soybeans was very low in comparison to the others. It appears that this is because much more short-chain fatty acids were produced in the cecal contents of the fermented, defatted soybean group than in the other groups, and there was active propagation of the intestinal bacteria.

Moreover, it is clear that the amount of cecal contents was significantly higher than in the other groups. The reason for this appears to be as follows:

The fermented, defatted soybeans are produced by koji preparation with untreated defatted soybeans using koji mold and then hydrolyzing the product. Regardless of whether the starting materials are the same, it appears that the increase in cecal contents, that is, undigested product, with fermented, defatted soybeans that have been obtained by the decomposition of untreated, defatted soybeans with the enzyme of koji mold is due to the fact that enzyme undecomposed product ( = undigested product) has been produced during the fermentation process with the fermented, defatted soybeans. That is, the increase in undigested product by administration of fermented, defatted soybeans indicates that the nutrients (undigested product) will quickly take effect on bacteria normally present in the digestive tract when fermented soybeans are administered. Furthermore, propagation of lactic acid bacteria, which are bacteria that are normally present in the digestive tract during fermentation of soybeans, can only mean that the lactic acid bacteria can increase using the enzyme undecomposed product of fermented, defatted soybeans in the intestines as the nutrient source after fermented, defatted soybeans have been administered.

On the other hand, when the Comparative Example is discussed, Watanabe et al. (*Nikon Eiyo Shokuryo Gakkaishi*, vol. 48, No. 4, 283-289 (1995)) report that there was an effect on rat growth and cecal flora in each group treated with casein, fermented soybeans and cooked soybeans. Cecal pH was somewhat lower than in the casein treatment group, but there was not a difference between the fermented soybeans and the cooked soybean groups.

The fact that the ability to produce short-chain fatty acids intestinally of fermented soybeans, which are a fermented product, is weak in comparison to fermented, defatted soybeans that have been fermented by koji mold can be derived by comparison with the above-described test findings of the inventors. The reduction in cecal pH with fermented, defatted soybeans when compared to untreated soybeans is due to the promoting effect on the propagation of lactic acid bacteria normally present in the digestive tract.

Consequently, addition of lactic acid bacteria and bifidobacteria, which are bacteria normally present in the digestive tract that are beneficial to humans and animals, to promote propagation during the production of fermented, defatted soybeans makes it possible to produce preparations of lactic acid bacteria that could not be made until now.

### Test 8

### Studies of resistant starch

### Object

The object is to confirm whether or not lactic add bacteria of fermented, defatted soybeans propagate in the presence of commercial undigested product preparations (resistant amylose: Yamanouchi Pharmaceutical Co., Ltd., brand name "Hi-Maize").

### Test method

In order to determine whether or not lactic acid bacteria propagate when fermented, defatted soybean (AO) is combined with undigested product preparation, the following four types were hydrolyzed to a water content of 75% by weight and the number of lactic acid bacteria and changes in pH after cultivation for 24 hours at 42°C were determined and compared.
1) Fermented, defatted soybean (AO) only
2) Addition of 0.1% by weight fermented, defatted soybeans to Hi-Maize (corn starch-derived)
3) Addition of 0.1% by weight fermented, defatted soybeans to corn starch (Tohkan Co., Ltd.)

In this case, the fermented, defatted soybeans are the bacteria preparation; and since the number of viable bacteria is as high as 10⁹/g, there is no need to administer large doses.

### Results

Changes in the number of lactic acid bacteria and pH of each type are shown in Table 11.

**Table 11**

| | | 0 hours | 24 hours |
|---|---|---|---|
| Corn starch | pH | 4.19 | 4.12 |
| | Number of lactic acid bacteria | 1.5 × 10⁵ | 1.4 × 10⁴ |
| Himaze | pH | 5.27 | 4.37 |
| | Number of lactic acid bacteria | 2.5 × 10⁵ | 9.6 × 10⁷ |
| Fermented soybeans | pH | 5.61 | 5.72 |
| | Number of lactic acid bacteria | 1.6 × 10⁶ | 3.9 × 10⁹ |

According to Table 11, the reduction in pH is the most marked and the increase in the number of lactic acid bacteria is marked with Hi-Maize (resistant amylose). The fact that there is a marked increase in lactic acid bacteria and a marked reduction in pH of fermented, defatted soybeans with Hi-Maize (resistant amylose) indicates that short-chain fatty acids are produced by lactic acid bacteria when undigested amylose is used as the nutrient.

Nevertheless, there was not an increase in lactic add bacteria with corn starch having a low undigested content, confirming the obvious difference between the two.

Consequently, by combining fermented, defatted soybeans (containing lactic acid bacteria) and resistant starch, such as Hi-Maize (resistant amylose), etc. with a high undigested component content, inhibiting effects on propagation of *Salmonella* bacteria in the intestines of animals, etc., can be expected because short-chain fatty acids are reliably produced.

Moreover, when defatted soybeans produced as described above are used as feed, etc., the defatted soybeans produced as in each of the above-described embodiments are dried and then pulverized so as to obtain pulverized defatted soybeans as in Figure 2.

As seen from the above, according to the present invention, it is possible to propagate live koji mold and remove the phytic acid from grains and reduce the molecular weight of the product even further by hydrolysis, thus providing a beneficial microorganism propagation-promoting material using the product. A beneficial microorganism propagation-promoting material that promotes propagation of beneficial microorganisms that are effective in maintaining the health of living beings during their rearing can be easily obtained. Moreover, the production process is simple and production cost is low.

In addition, a product that more effectively absorbs minerals can be obtained by eliminating phytic acid via the liberation of at least 2 phosphoric acid groups from phytic acid.

Furthermore, though the above embodiments are described with reference to the present invention used for defatted soybeans whose main component is proteins, the present invention can also use rice, etc. whose main component is starches, etc., as the grain; and it further can use a mixture of rice, etc., and defatted soybeans. A combination of grains containing saccharides, such as oligosaccharides, and another type of grain can also be used. Moreover, the present invention is likewise suitable for any product that uses grains containing phytic acid as the starting material, that is, from human foods to feed used for cultivation and breeding.

In addition, the present invention is very practical because it can be executed using a conventional koji-preparation device as is and does not require the special production of equipment with an industrial base.

The present invention is not limited to the above-described embodiments, and modifications can be implemented as needed.

## Claims

1. A beneficial microorganism propagation-promoting material which promotes propagation of a beneficial microorganism, said material being obtained by inoculating koji mold on grains to effect koji preparation, adding water to a resultant from said koji preparation to thereby hydrolyze proteins and/or saccharides contained in said resultant, and removing a predetermined amount of phytic acid contained in said grains.

2. A beneficial microorganism propagation-promoting material comprising a mixture of:
a product for promoting a propagation of beneficial microorganisms obtained by inoculating koji mold on grains to effect koji preparation, adding water to a resultant from said koji preparation to thereby hydrolyze proteins and/or saccharides contained in said resultant, and removing a predetermined amount of phytic acid contained in said grains; and
resistant starch.

3. A beneficial microorganism propagation-promoting material according to Claim 1 or 2, wherein propagation of a beneficial microorganism contained in said resultant from said koji preparation and/or a beneficial microorganism added to said resultant from said koji preparation is promoted during said hydrolysis.

4. A beneficial microorganism propagation-promoting material according to Claim 1, 2 or 3, wherein said beneficial microorganism is at least one selected from *Eumycetes*, lactic acid bacteria and bifidobacteria.

5. A process for preparing a beneficial microorganism propagation-promoting material which promotes propagation of a beneficial microorganism, said process comprising: inoculating koji mold on grains to effect koji preparation, adding water to a resultant from said koji preparation to thereby hydrolyze proteins and/or saccharides contained in said resultant, and removing a predetermined amount of phytic acid contained in said grains.

6. A process for preparing a beneficial microorganism propagation-promoting material according to Claim 5, wherein propagation of a beneficial microorganism contained in said resultant from said koji preparation and/or a beneficial microorganism added to said resultant from said koji preparation is promoted during said hydrolysis process.

7. A process for preparing a beneficial microorganism propagation-promoting material according to Claim 5 or 6, wherein said beneficial microorganism is at least one selected from *Eumycetes*, lactic acid bacteria and bifidobacteria.
